# EUROPEAN PATENT APPLICATION

(11) **EP 4 201 191 A1**
(43) Date of publication of application: **28.06.2023**
(21) Application number: 22215427.0
(22) Date of filing: 21.12.2022
(51) Int. Cl.: A01D 46/00, A01D 46/20, A01D 46/30

(54) **FRUIT PICKING ROBOTIC INSTALLATION ON PLATFORMS**

(30) Priority: 21.12.2021 US 202163292011 P
(71) Applicant: TEVEL AEROBOTICS TECHNOLOGIES LTD, 7174910 Modiin (IL)
(72) Inventor: MAOR, Yaniv, Modiin (IL)
(74) Representative: Balder IP Law, S.L.

(57) **Abstract**

The present invention provides a robotic harvesting platform system for harvesting or diluting fruits in an orchard, the system comprising an elongated platform attached to a support frame, and two or more robotic harvesting units associated therewith at different points thereon.

## Description

### FIELD OF THE INVENTION

The present invention is in the technical field of agriculture equipment, specifically harvesting. More particularly, the present invention relates to harvesting-, dilution- and pruning-devices for orchards, plantations and green houses, such as apple-, pear-, apricot-, peach-, orange-, small-citrus fruit-, and lemon-trees, avocado, vines, tomatoes, eggplants, cucumbers, and peppers.

### BACKGROUND OF THE INVENTION

The growing season is a steady race to harvest- after a long period of time of preparation, planning, pruning, thinning, etc., the entire bottom line comes to the days of harvest, which are often very limited to obtain all fruits when they're ripe and before they are too ripe. Accordingly, orchard equipment have been developed to improve efficiencies and reducing orchard harvest costs.

Manual labor using ladder harvest is an inefficient act, no matter how fast the pickers are: a picker spent a lot of time to climb down the ladder, walk over to the bin, dump the fruits therein, walk back to the tree, move the ladder to the next tree, climb up the ladder and start the process again.

Conventional orchards harvesting devices are based on mass labor work and supportive tools, like automatic secateursor that is held by and operated by humans, or automatic ladders (as described in US 3,568,796). The advanced tools are large tracks with a few robotic arms. Such tracks, as developed by *HRA Laboratory* are large, expensive and complicated. Each robotic arm needs to be long and flexible enough to pick from the trees top or bottom. The robotic arms need to have at least 4 degrees of freedom, which causes very expensive solutions.

Various attempts have been made to improve such robotic harvesting. For instance, ZhaoDe-An et al. (Biosystems Engineering, 2011, Vol. 110(2): 112-122) provides a robotic device for harvesting apples using a vision-based module and fruit recognition algorithmonce the device identifies a fruit, a robotic arm is extended to harvest it. Jia et al. (International J. of Advanced Robotic Systems, 2020: 1-16) provides an architecture of a motorized apple harvesting robot having a harvesting arm.

US 2013/0204437 describes an automated agricultural robot system and method of harvesting fruits using said robot. The robot comprises a harvesting arm that can be extended to reach all areas in the tree to harvest remote fruits.

WO 2008/008972 describes an autonomous robotic picker having multiple hollow harvesting arms that can move in any direction for harvesting and delivering the fruits within the hollow arms into a fruit collection bin at the base of the picker.

Contrary to the present invention, all known robotic harvesters have a long harvesting arm(s) that is designed to reach all the way to the treetop. Such long arms are complicated, hard to control, require massive engines and consume a lot of energy. Due to their length, such long arms have long vertical degree of freedom which both reduces accuracy and efficiency since long movements are required. In addition, long arms are expensive, and due to their size only a limited number of arms can be installed on each platform.

Accordingly, a need exists for a robotic harvester that overcome all of the deficiencies of the known robotic harvesters.

### SUMMARY OF THE INVENTION

The present invention provides a robotic harvesting platform system for harvesting or diluting fruits in an orchard, the system comprising: (i) a support frame; (ii) an elongated platform attached to said support frame; (iii) two or more robotic harvesting units associated with said elongated platform at different point thereon; and (iv) one or more fruit detection units for identifying location and position of said fruits, wherein: said robotic harvesting platform system is designed to harvest fruits from a tree(s) at different heights simultaneously; and each robotic harvesting unit is designed to operate independently from the other robotic harvesting units.

### BRIEF DESCRIPTION OF THE DRAWINGS

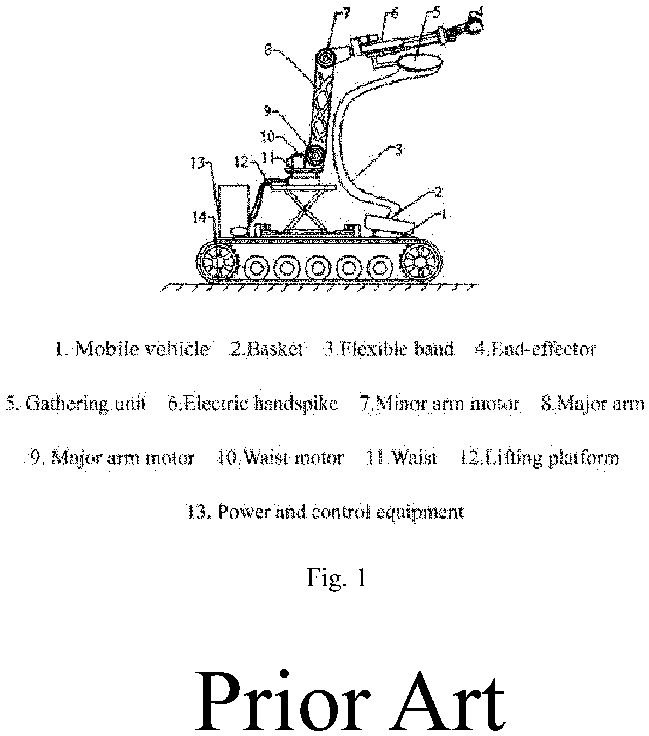

**Fig.** 1 is a diagram of the apple robotic harvester of Jia, 2020.
**Figs. 2A-2C** are schematic illustrations of robotic harvesting platform systems according to some embodiments of the invention.

### DETAILED DESCRIPTION OF THE INVENTION

Many harvesting robots have been developed comprising means for transporting on the ground of an orchard, a fruit collection bin and one or more fruit harvesting arms. Due to the height of the trees, the harvesting arms are constructed to enable harvesting fruits even at the top of the tree, e.g. by being extractable or having multiple joints that when spread open provide the necessary length. However, such arms are cumbersome and tend to malfunction due to their complexed structure and load generated when they are fully extended.

The present invention is aimed at providing a platform equipped with multiple harvesting units, each unit is located at a different location on the platform, such that when the platform elevates in an angle, each harvesting unit is positioned at a different height relative to the other units thereby enabling reaching various heights of the tree simultaneously while using simple and short-length harvesting units/arms.

Accordingly, the present invention provides a robotic harvesting platform system for harvesting or diluting fruits in an orchard, the system comprising: (1) a support frame; (2) an elongated platform attached to said support frame; (3) two or more robotic harvesting units associated with said elongated platform at different point thereon; and (4) one or more fruit detection units, such as a camera, for identifying location and position of said fruits, wherein: (i) said robotic harvesting platform system is designed to harvest fruits from a tree(s) at different heights simultaneously; and (ii) each robotic harvesting unit is designed to operate independently from the other robotic harvesting units and is deigned to harvest fruits independently therefrom.

In certain embodiments, each harvesting unit is equipped with its own fruit detection unit. In alternative embodiments, a single fruit detection unit is used for several harvesting units. In further alternative embodiments, each harvesting unit is equipped with its own fruit detection unit, and one or more fruit detection units are used for several harvesting units. The term "used for a harvesting unit" means that data received from a fruit detection unit is used, e.g. by a computing system, to guide the harvesting unit to a fruit.

In certain embodiments, the robotic harvesting platform system of the invention further comprises any one of: (i) one or more sensors for collecting various fruits' data, such as a camera that measures the size, color and shape of a fruit, and optionally a device that have tactile feedback about the fruit softness. Fruits' data may include quality, defects, ripeness, color, size, position, etc. or any combination thereof; (ii) a fruit conveyer and fruit collection bin- once a fruit is harvested, it is placed onto the conveyer and is transported to the fruit collection bin; (iii) a fruit sorter designed to sort harvested fruits, e.g., according to fruit's quality, size, shape, etc., or any combination thereof; and (iv) a computing system comprising a processor and memory, adapted for controlling said robotic harvesting platform system and said two or more robotic harvesting units, or any combination thereof.

In certain embodiments of the robotic harvesting platform system of any of the embodiments above, the one or more fruit detection units is further adapted for collecting various fruits' data (quality, ripeness, color, size, position, etc.). Such fruits' data can be transmitted to a computing system (optionally the integral computing system in the robotic harvesting platform system), which then use the data to determine whether a fruit needs to be harvested or not and/or whether a fruit is damaged and need to be disposed, and/or determine a fruit's quality and sort it accordingly.

In certain embodiments of the robotic harvesting platform system of any of the embodiments above, when the robotic harvesting platform system comprises a computing system, said computing system is designed to synchronize platform's driving and movement and harvesting units' (103) activation, such that when all the fruits have been harvested, the platform is moved forward to the next area/tree to continue harvesting.

In certain embodiments, the one or more sensors and/or one or more fruit detection units used in the robotic harvesting platform system according to any of the embodiments above is selected from any one of: one or more cameras, lasers, eco/sound sensors, etc.

In certain embodiments, the harvesting platform system according to any of the embodiments above further comprises a vision system, optionally as part of said one or more fruit detection units, which is designed to lock onto a fruit, and together with the computing system, control the movement of the platform system and its harvesting units until grasping the fruit (or its stipe).

In certain embodiment of the harvesting platform system according to any of the embodiments above, the computing system comprises a memory and processor adapted for controlling said robotic harvesting platform system and said two or more robotic harvesting units. In specific embodiments, the computing system is designed to receive data, e.g. from said one or more sensors and one or more detection units, regarding the amount and/or quality of the fruits, orchard plan/map, etc. The computing system may further be configured to guide the robotic harvesting platform system in the orchard based on, e.g., visual and other sensory elements that helps guiding it and avoid collision with obstacles, and/or an orchard map.

Accordingly, in certain embodiments, the harvesting platform system according to any of the embodiments above is further equipped with an anti-collision system, which prevents unintentional collision thereof with trees, people and other systems, and enables safe navigation in a complex environment, e.g. an orchard.

In further embodiments, the computing system further comprises an algorithm for determining fruit's quality based on data received from said one or more sensors. In certain embodiments, said algorithm for determining the fruit's quality uses at least one of the following parameters for determining the fruit's quality, including ripeness, according to the type of fruit being harvested: color, water content, rigidity/softness, sparkle, size, season, spots-damages inspection, fruit disconnection force (the ripper the fruit is- the easier it is to pull), weight.

In further embodiments, the computing system further comprises an algorithm that determines fruit's position and instruct/navigates the harvesting units to an optimal harvesting position, and optionally also an algorithm that decides if the fruit is ripe and ready to be plucked.

In certain embodiments, the harvesting platform system according to any of the embodiments above is driven by an operator through said orchard. Alternatively, the harvesting platform system is configured to autonomously navigate through the orchard using, e.g., an integrated computing system. In certain embodiments, the harvesting platform system according to any of the embodiments above is self-driving capable, namely it can drive and maneuver autonomously.

In certain embodiments of the harvesting platform system according to any of the embodiments above, the: (a) large horizonal movement of the harvesting units (103) is done by horizonal movement of the entire platform; (b) large vertical movement of the harvesting units (103) is not required (since each harvesting unit is associated with the platform at a different height); and (c) small vertical and horizontal movements of the harvesting units (103) are done the harvesting units (103) themselves.

In certain embodiments, the computing system enables the robotic harvesting platform system of the invention to harvest fruits in an independent/autonomous manner so that there is no need for manual harvesting or manual control of the harvesting process. This independency is obtained, e.g., by automatically identifying the fruits for harvesting, autonomously maneuvering each one of the two or more robotic harvesting units the to the fruit(s) to be harvested based on data obtained from said one or more fruit detection units, autonomously select a fruit(s) for picking, etc., or any combination thereof.

In certain embodiments, the computing system is designed to enable the robotic harvesting platform system of the invention to be completely independent/autonomous so that there is no need for a manual control- both for maneuvering in the orchard and for the harvesting process. This independency is obtained, e.g., by automatically maneuvering the platform in the orchard while harvesting, autonomously navigate each one of the in a complex environment, autonomously avoiding obstacles, autonomously maneuvering each one of said two or more robotic harvesting units the to the fruit(s) to be harvested based on data obtained from said one or more fruit detection units to the fruit to be harvested based on data obtained from a fruit detection unit, autonomously select a fruit(s) for picking, etc. or any combination thereof.

In certain embodiment of the harvesting platform system according to any of the embodiments above, the support frame is, or constitutes part of, a motorized vehicle designed to move in an orchard. In such a case, and when the system is autonomous, the computing system is further designed to control the maneuvering of the harvesting platform system in the orchard. In alternative embodiments, the support frame is designed to be mounted onto a motorized vehicle. In such alternative embodiments, the motorized vehicle might be manually operated or may be automatic, in which case the computing system of the harvesting platform system may be designed to further interact with the motorized vehicle's systems and control its movement in the orchard.

In certain embodiments, the harvesting platform system according to any of the embodiments above, further comprises a positioning unit (GPS or LPS or ultra-wide-band or visual positioning system).

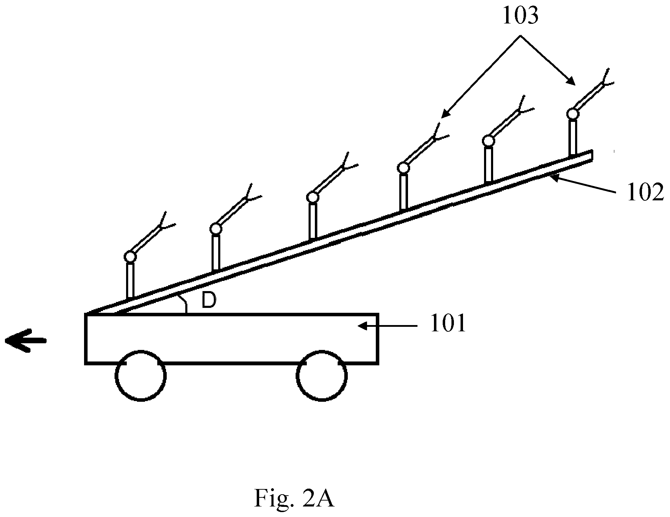

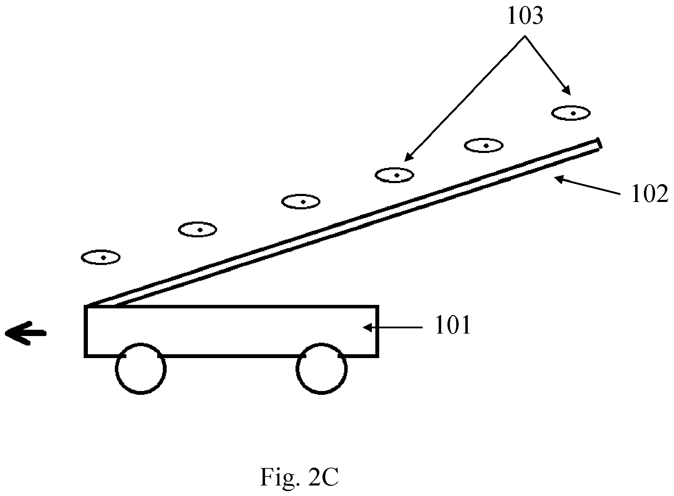

In certain embodiments of the robotic harvesting platform system of any of the embodiments above, the elongated platform is extractable meaning that it can be extended such that the robotic harvesting units associated therewith are spaced apart from one another as the platform is extracted and get closer as it is shortened. In a further embodiment, the elongated platform is liftable, meaning that it can be raised and lowered according to need thereby enabling placing each of the robotic harvesting units at a desired height according to the trees' height in the orchard, thereby reducing the maneuvering required by each robotic harvesting unit to reach the fruits. In specific embodiments, the lifting of the platform is carried out in an angle relative to the support frame, such that the harvesting unit located closest to the support frame is at a lower height relative to the harvesting unit located remotely therefrom (i.e. the harvesting unit located at the most remote point from the support frame is the highest one). See Figs. 2A-2C that illustrate the different heights of the harvesting units along the platform.

The extractability and lift-ability of the platform is beneficiary in that it enables easier storage of the robotic harvesting platform system when not in use, as well as easier transportation from place to place, such as into and out of an orchard.

In certain embodiment of the harvesting platform system according to any of the embodiments above, the robotic harvesting units are autonomous harvesting arms physically connected to the platform, said arms are designed to move in any direction. In alternative embodiments, the robotic harvesting units are autonomous unmanned aircraft vehicle (UAV) connected to the platform via a power line. In further alternative embodiments, some of the robotic harvesting units are harvesting arms and some are UAVs.

In both cases, namely when the harvesting unit is either a harvesting arm or a UAV, it comprises a robotic arm having a length sufficient to protrude the branches and accessing the fruits within, while being short enough to avoid cumbersomeness and/or un-balancing of the UAV. The arm may be a flexible arm with hinges providing the arm with at least 2 degrees of freedom, which enables easy access to the fruit without too much maneuvering. Notably, in the case of a UAV, the arm may be rigid, and the degrees of freedom is obtained by the movement of the UAV itself. Any type of arm can be used, such as a gripper (e.g. forceps, clamps, or robotic fingers), a pulling arm, a suction nipple, or any combination thereof. The plucking of the fruits off a tree can be done by any fruit-cutting mechanism, such as by pulling, twisting, cutting (using a dedicated cutter, trimmers, wire loops, secateurs, saw, scissors, shears, etc.), or any combination thereof.

In certain embodiments, the robotic harvesting platform system of any of the embodiments above comprises 2-10 robotic harvesting units. The number of the harvesting units is determined according to need, the size of orchard, trees' size and height, number and type of fruits on each tree, etc. The distance between two adjacent robotic harvesting units is either the same or different along the platform. The distance between two adjacent robotic harvesting units can be adjusted according to need: for instance, when the elongated platform has a fixed length, the distance between two adjacent robotic harvesting units can be determined in advance by attaching each harvesting unit at predefined position thereby determining the distance between the units. Alternatively, or in addition, when the length of the elongated platform is adjustable, i.e. it can be extracted and shortened, the distance of the harvesting units can be actively adjusted according to need, i.e. by extracting and shortening the elongated platform itself.

The harvesting units are designed to move in any direction to reach fruits from any angle. The fact that they are associated with the platform and are positioned thereby at a certain height means that they do not require long and cumbersome crane (in case of a harvesting arm) or power cord (in case of a UAV), which makes the overall construction of the harvesting system cheaper and easier to maintain, handle and operate. Positioning/ raising the harvesting units at different levels/heights means that there is a harvesting unit in close proximity to the fruits at any level/height on the tree, rendering the need of a long and expandable harvesting arm moot. Another advantage of a shorter harvesting arm/ distance means that the harvesting unit can harvest faster since it does not need to move long distances back & forth and up and & down from the tree to the collection bin, thereby shortening harvesting time per fruit and overall orchard's harvesting time.

In certain embodiment of the harvesting platform system according to any of the embodiments above, the conveyor is located on the platform, between the different harvesting units, thereby further shortening harvesting time by enabling the harvesting units to place the harvested fruit on the nearby conveyor instead of in a remote collection bin. The conveyor may be a simple slope in which fruits roll by gravity, or may be a motorized, e.g., belt that actively pulls the fruits towards the collection bin (and optionally via a sorting unit).

In certain embodiments, the harvesting units are installed onto the conveyor. In specific embodiments of the harvesting platform system according to any of the embodiments above, the platform consists essentially entirely of the conveyor.

In certain embodiments, the present invention provides a robotic harvesting platform system for harvesting or diluting fruits in an orchard, the system comprising: (1) a support frame (101); (2) an elongated platform (102) attached to said support frame, the platform comprising or consisting of a conveyor, wherein the platform is extractable and liftable; (3) two or more robotic harvesting units (103) associated with the elongated platform (102), wherein the units are either harvesting arms or UAV, or both; (4) one or more fruit detection units, such as a camera, for identifying location and position of said fruits, and optionally for collecting various fruits' data (quality, ripeness, color, size, position, etc.). that can be used by the computing system to determine whether a fruit needs to be harvested or not and/or whether a fruit is damaged and need to be disposed, and/or determine a fruit's quality and sort it accordingly; (5) a fruit conveyer and fruit collection bin, such that once a fruit is harvested by the harvesting unit(s), it is placed onto the conveyer and is transported / rolls to the fruit collection bin; and (6) a computing system comprising a processor and memory, adapted for controlling said robotic harvesting platform system and said two or more robotic harvesting units, and optionally further adapted to receive data, e.g. from said one or more sensors and one or more detection units, regarding the amount and/or quality of the fruits, orchard plan/map, etc., and guide the robotic harvesting platform system in the orchard, wherein: (i) said robotic harvesting platform system is designed to harvest fruits from a tree(s) at different heights simultaneously; and (ii) each robotic harvesting unit is designed to operate independently from the other robotic harvesting units and is deigned to harvest fruits independently therefrom.

In specific embodiment thereof, the computing system enables the robotic harvesting platform system of the invention to harvest fruits in an independent/autonomous manner so that there is no need for manual harvesting or manual control of the harvesting process, by, e.g., automatically identifying the fruits for harvesting, autonomously maneuvering each one of the two or more robotic harvesting units the to the fruit(s) to be harvested based on data obtained from said one or more fruit detection units, autonomously select a fruit(s) for picking, etc., or any combination thereof, and optionally by automatically maneuvering the platform in the orchard while harvesting.

In certain embodiments, the above robotic harvesting platform system further comprises: (i) one or more sensors for collecting various fruits' data, such as a camera that measures the size, color and shape of a fruit, and optionally a device that have tactile feedback about the fruit softness. Fruits' data may include quality, defects, ripeness, color, size, position, etc. or any combination thereof; and/or (ii) a fruit sorter designed to sort harvested fruits, e.g., according to fruit's quality, size, shape, etc., or any combination thereof.

The present invention further provides a method for harvesting fruits in an orchard, the method comprising the steps of: (i) providing a robotic harvesting platform system according to any of the embodiments above; (ii) positioning same near a tree and extending the elongated platform in accordance with the tree's height; (iii) activating the two or more robotic harvesting units, thereby enabling autonomous harvesting thereby, wherein each robotic harvesting unit is designed to harvest fruits at a different height of the tree independently from one another; and (iv) once picking is complete for a certain tree, moving the robotic harvesting platform system to the next tree, and so forth, e.g. until all fruits in the orchard have been harvested.

Modern orchard trees are usually at a height of from about 2 to about 5 meters. As a result, when working with 5-meters trees, the long arm movement or UAV movement requires a movement range of at least 5 meters (from tree top to a collection bin on the ground), which is time consuming. If assuming that the speed of a robot movement is about 1 meter/sec, then the time needed for the harvesting arm to move backwards and forwards is about 5 seconds (2x2.5). The use of a platform of the invention enables to reduce this height by at least half, depending on the number of harvesting units (103) used/mounted on the platform: for instance, if 10 harvesting units (103) are installed, the distance each unit would need to move will be less than 1/10 of the tree's height, namely 0.5 meter for a 5-meter tree, and the time required for such a movement would be about 0.5 second, which saves about 4.5 seconds per fruit- this is over 90% time saving!

The invention will now be illustrated by reference to the accompanying drawings which are to be considered only as representative examples of possible embodiments of packages of the invention.

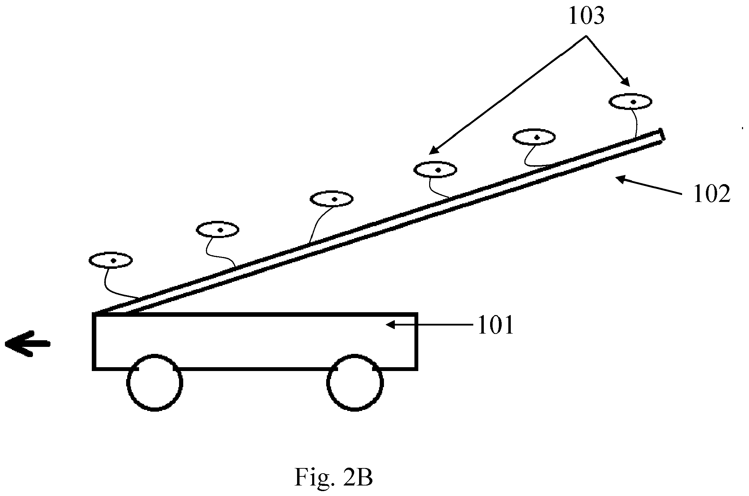

Figs. 2A-2C illustrate 3 different possibilities of robotic harvesting platform systems according to the invention, showing the support frame (101), which is illustrated as a wheeled vehicle, the elongated platform attached thereto (102) and the harvesting units (103), which are either harvesting arms (Fig. 2A), harvesting wired UAVs connected to the platform via a wire (Fig. 2B), or harvesting independent UAVs associated with the platform, e.g., via positioning units. In specific embodiments, when the harvesting units (103) are UAVs, each UAV identifies its location relative to the platform by, e.g. a camera facing down to the platform and/or with a (ultrasonic) positioning sensor and optionally by placing one or more markers on the platform that can be detected by the sensor and/or camera.

As can be seen, the platform (102) is elevated to an angle (D) relative to the support frame (101). Each harvesting unit (103) constitutes a region-of-interest (ROI), which is defined by the height divided by the number of harvesting units installed. For example, if the trees in the orchard are 4 meters tall, and the number of harvesting units is 4, then the ROI equals to 1 meter (i.e. 4 divided by 4), with some overlaps between the harvesting units.

In certain embodiments, the ROI according to the invention is up to 2 meters, up to 1.5 meter or up to 1 meter. Notably, in known harvesting robots today, the ROI is usually 3-4 meters.

## Claims

1. A robotic harvesting platform system for harvesting or diluting fruits in an orchard, the system comprising:
i) a support frame;
ii) an elongated platform attached to said support frame;
iii) two or more robotic harvesting units associated with said elongated platform at different points thereon; and
iv) one or more fruit detection units for identifying location and position of said fruits,
wherein:
- said robotic harvesting platform system is designed to harvest fruits from a tree(s) at different heights simultaneously; and
- each robotic harvesting unit is designed to operate independently from the other robotic harvesting units.

2. The robotic harvesting platform system of claim 1, further comprising one or more sensors for collecting various fruits' data.

3. The robotic harvesting platform system of claim 1, wherein said one or more fruit detection units is further adapted for collecting various fruits' data.

4. The robotic harvesting platform system of claim 1, further comprising a fruit conveyer and fruit collection bin.

5. The robotic harvesting platform system of claim 4, further comprising a fruit sorter designed to sort harvested fruits. According to fruit's quality, size, shape, etc., or any combination thereof.

6. The robotic harvesting platform system of claim 1, further comprising a computing system comprising a processor and memory, adapted for controlling said robotic harvesting platform system and said two or more robotic harvesting units, as well as to synchronize platform's driving and movement and harvesting units' (103) activation.

7. The robotic harvesting platform system of claim 1, wherein said system is driven by an operator through said orchard.

8. The robotic harvesting platform system of claim 1, wherein said system is configured to autonomously navigate through said orchard.

9. The robotic harvesting platform system of claim 1, which is self-driving capable.

10. The robotic harvesting platform system of claim 1, wherein:
a) large horizonal movement of the harvesting units (103) is done by horizonal movement of the entire platform;
b) large vertical movement of the harvesting units (103) is not required (since each harvesting unit is associated with the platform at a different height); and
c) small vertical and horizontal movements of the harvesting units (103) are done the harvesting units (103) themselves.

11. The robotic harvesting platform system of claim 1, wherein said support frame is a motorized vehicle designed to move in an orchard.

12. The robotic harvesting platform system of claim 1, wherein said support frame is designed to be mounted onto a motorized vehicle.

13. The robotic harvesting platform system of claim 1, wherein said elongated platform is extractable and liftable.

14. The robotic harvesting platform system of claim 1, wherein said robotic harvesting units are autonomous harvesting arms physically connected to the platform, said arms are designed to move in any direction.

15. The robotic harvesting platform system of claim 1, wherein said robotic harvesting units are autonomous unmanned aircraft vehicle (UAV) connected to the platform via a power line.
